# EUROPEAN PATENT APPLICATION

(11) **EP 0 590 563 A1**
(43) Date of publication of application: **06.04.1994**
(21) Application number: 93115556.8
(22) Date of filing: 27.09.1993
(51) Int. Cl.: C07D 495/04, A61K 31/415

(54) **Biotin amides, a process for their preparation and therapeutical compositions containing them as the active principles for the treatment of diabetes and relative complicating diseases**

(30) Priority: 28.09.1992 IT MI922232
(71) Applicant: LIFEGROUP S.p.A., I-35043 Monselice (Padova) (IT)
(72) Inventor: Della Valle, Francesco, I-35122 Padova (IT); Lorenzi, Silvana, I-35142 Padova (IT); Marcolongo, Gabriele, I-35020 Carrara San Giorgio, Padova (IT); Galzigna, Lauro, I-35142 Padova (IT)
(74) Representative: Moretti, Giorgio

(57) **Abstract**

Biotin amides, a process for their preparation and therapeutical compositions containing them as the active principles for the treatment of insulin-independent and insulin-dependent diabetes and relative complicating diseases, such as peripheral neuropathies, chronic and invalidating pathologies.

## Description

### FIELD OF THE INVENTION

The present invention refers to biotin amides, a process for their preparation and relative therapeutic compositions for the treatment of diabetes and its complicating diseases.

### PRIOR ART DISCLOSURE

The clinical role of biotin, better known as vitamin H, was determined in 1942 by Sidenstricker and coworkers, who demonstrated that the so called "egg white injury" could be eradicated, by administering biotin.

Before, other researchers had indeed identified this syndrome in animals subjected to a diet consisting only of egg white, and it could be prevented by biotin administration.

Sidenstricker's experiment was directed to demonstrate which were the consequences caused by vitamin H deficiency in human organism.

In fact in patients subjected to a diet of egg white, in which biotin results to be linked to a protein, avidin, not rendering bioavailable vitamin H, a biotin deficit state was observed.

As it clearly resulted from this experiment and from the following ones, this deficit was associated with clinical phenomena, which depending on the seriousness and on the symptoms ranged from non pruriginous dermatitis to maculo-squamous dermatitis, to disorders such as mental state alterations, myalgia, hyperesthesia,localized paresthesia, anorexia, coronary ischemia.

All these secondary phenomena disappeared and the patients returned to normality after a prolonged biotin administration for a determined time period.

In the same years also the biologic role of biotin was found, Vitamin H acted as a co-factor in carboxylation enzymatic reactions. In this connection in the last 20 years importance has been ascribed in applying enzymatic reactions co-factors for the therapy of human diseases.

Succesively it was demonstrated that biotin because of its peculiarity as a cofactor in enzymatic reactions could be advantageously used in therapy in the same way as pyridoxine and vitamin D.

For example it was found that, whereas biotin proved uneffective for the treatment of diseases caused by single carboxylase enzymes insufficiencies, such as the propionyl- CoA carboxylase deficit (which is the cause of ketotic hyperglycinemia), pyruvate-carboxylase deficit (which is the cause of lactic acidosis persisting during the various chilhood phases), β-methyl-crotonylcarboxylase deficit (which may also cause in childish age spinal and muscular atrophy or again in childish age also a serious dysmetabolic acidosis), on the contrary biotin resulted effective for the treatment of diseases caused by multiple carboxylase enzymes deficiencies, whose symptoms are more or less common to those of the above mentioned pathologies and caused by single carboxylase enzymes deficiencies.( K.S.Roth "Biotin in clinical medicine - a review", " The American Journal of clinical nutrition" 34: September 1981, pp 1967-1974).

Besides, Vitamin H proved particularly effective in the treatment of newborn seborrheal dermatitis, whose cause has not yet been well ascertained, but seeming to be ascribable to low content of vitamin H in mother's milk, defective digestion or persistent diarrhea (Krishnamurti- Dachshinamurti and Jasbir Chauan-Biotin-Vitamins and hormones vol.45 -1989 pp 337-385).

The evidences concerning a possible involvement of biotin in conditions of glucose altered metabolism began in 1968, when the ability of biotin to restore the activity of a key glycolytic enzyme, glucokinase, was demonstrated (Dachshinamurti K., Cheah-Tan C., 1968,Arch.Biochem. & Biophys.,127: 17-21). This substance is in fact the catalyst of the initial step of the glucose utilization by the cell, therefore it is the enzyme mostly involved in the control of glucose hepatic uptake.

It is in fact known that glucokinase enzyme regulates hematic glucose, namely glycemia, contemporaneously controlling the utilization and storing of hepatic glucose in the form of glycogen, and in pancreas islets glucokinase is considered to act as a glucose sensor, regulating insulin production.

The effect of biotin on hepatic glucokinase resulted to be dependent from the genic regulation of this enzyme and mediated by mRNA induction for the same enzyme, therefore an analogous behaviour to that of insulin (Chauan J. et al. J. Biol Chem., 1991, 266, 10025-10038).

In addition biotin, thanks to its co-factor role, may have an important function in the control of the hematic glucose, also controlling an other enzymatic route.

Biotin is in fact able to activate as a co-factor in carboxylation reactions besides glucokinase, also pyruvate-carboxylase, namely the enzyme involved in gluconeogenesis; this activity is only apparently in contrast with glycolysis activity stimulated by glucokinase activation, since, indeed, pyruvate carboxylase is a key enzyme in the activation of Krebs cycle, an activation determining the use of hematic glucose and fat acids. Also acting on pyruvate carboxylase biotin can therefore cause a reduction of glycemia.

Later on also clinical evidences appeared correlating biotin levels to glycemia under diabetes mellitus conditions.

From the above observations one deduced that hyperglycemia typical of diabetes mellitus was ascribable to the increase of biotin in the tissue levels, since to this increase, a glucose increase corresponded.

In addition it was observed that, in a group of diabetic patients, once the insulin treatment was interrupted, the use of placebo caused a high increase of plasmatic glucose, whereas the use of biotin (16mg/die for 1 week) induced a significant reduction in the hematic glucose rate (Coggeshall, J.C. 1985, Life Sciences, NY Acad.Sci. 447,pp389-392).

Also in diabetes experimental models, as in the genetically diabetic mice KK being moderately hyperglycemic and insulin resistant diabetic non biotin deficient, high biotin doses resulted effective. In particular, the treatment with biotin (2mg/kg for 14 days), improved sensibly glucose tolerance of these animals (Reddi A. et al, 1988, Life Sciences, 42:pp 1323-1330).

More recently a very interesting evidence from the clinical point of view was issued, relating to the use of biotin not so much for glucose metabolism normalization as for the treatment of neuropathy, a complicating disease very frequently associated with diabetes (Koutsikos D., et al., 1990, Biomed & Pharmacother. 44: 511-514).

In fact the intramuscular treatment with biotin for 6 weeks associated with the oral administration of the same active principle at a daily dose of 5 mg for about 2 years- produced a marked improvement in the subjective neurologic symptoms (muscular cramps and paresthesiae, improvement in walking and in climbing stairs; disappearance of restless leg syndrome) as well as a slight improvement in conduction velocity at the level of the motor nerves and of the action sensory potentials.

The decrease in pyruvate-kinase activity, consequent to the decrease in biotin levels, according to the same authors should be the cause of neuropathic phenomena connected with diabetes, as well as of neuropathic symptomatologies associated with other dysmetabolic and toxic conditions, such as alcoholism and uremia. Notwithstanding the above described important results concerning the activity of biotin in models which can be correlated to diabetes, and in same diabetes, suggesting a wide use of biotin, alone or in association with antidiabetic drugs orally administered for the treatment of insulin-independent diabetes, or in association with insulin in case of insulin-dependent diabetes, no therapeutic use results, up to day to this purpose, of this important vitamin.

This is probably caused by its quick and non persistent action, rendering difficult the definition of a therapeutic and posology scheme.

On the other hand it is generally accepted that through a diet it is possible to have a sufficient biotin supply to cover physiological needs. In fact biotin is present in many and widely diffused aliments (milk, egg yolk etc.), and it is also produced by intestinal flora.

If this is true under normal conditions, it is however to be considered that in diabetic patients important alterations in the plasmatic levels of biotin have been observed and that only free biotin seems to regulate the use of hematic glucose.

In view of the foregoing it results particularly interesting, therapeutically speaking, to have biotin derivatives able to act as the vitamin as such, while possibly maintaining in the time the highest hematic levels. It is finally to be considered that biotin as such is poorly soluble in water and that limits its use by general parenteral route, said use being very important under serious diabetic states or in some extremely invalidating complicating diseases of its such as peripheral neuropathies.

Under these conditions it can be of particular therapeutic utility to obtain a quick bioavailability of this vitamin administered alone or in association with oral hypoglycemia drugs in case of insulin-independent diabetes or with insulin in case of insulin-dependent diabetes.

Therefore the need was felt to have completely water soluble biotin derivatives_{,} allowing the use of this active principle not only by oral, but also by parenteral and in particular by intramuscular and intravenous route.

### THE PRESENT INVENTION

The Applicant has now unexpectedly found that amide derivatives of α or β biotin having general formula (I):

W-CONH-R-X (I)

wherein W is 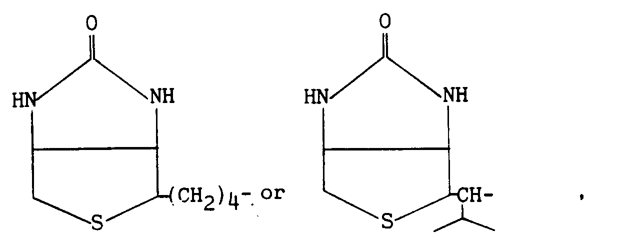
R is a bivalent radical of from 1 to 20 carbon atoms having the meanings defined in one of the following classes:
A) is a linear or branched alkylene radical, optionally substituted in the alkylene chain with at least one substituent selected from the group consisting of : -COOH, NH₂, 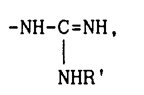 H, SH, -CO-, OH and wherein R' is methyl or H;
B) is a cycloalkylene of from 3 to 7 carbon atoms;
C) is an arylene or an arylalkylene radical optionally susbtituted on the aromatic ring with at least one NO₂ group;
D) is a piperazinylalkylene radical,
and X is selected from the group consisting of H, OH, COOH, H, 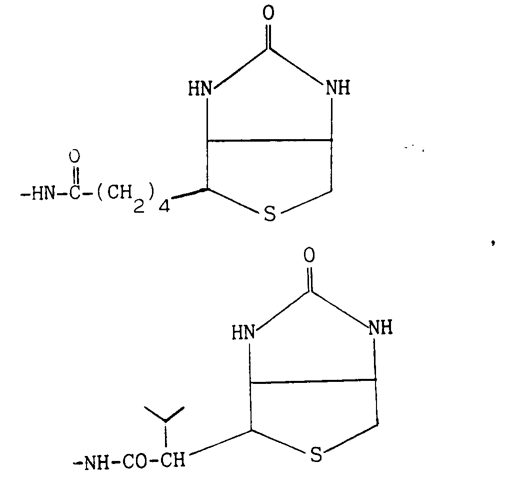
and
are able to act as hyperglycemia inhibitors, as well as to activate hepatic glucokinase in a higher degree than biotin as such.

Therefore the present invention relates to therapeutic compositions containing as the active principle at least one of the amides of general formula (I), in combination with suitable excipients and/or diluents, these compositions being in particularly suitable for the treatment of diabetes and for the treatment and prevention of its complicating diseases.

The amide derivatives of general formula (I) may be prepared according to two different processes .

The first process comprises the following steps:
a) treating biotin in a polar aprotic solvent, in the presence of a tertiary amine at a temperature lower than 0°C with alkylchloroformate (II):

   Cl-COOR'' (II)

   wherein R'' is a C₁-C₁₀ alkyl, thereby obtaining the corresponding mixed anhydride of formula (III);

   W-NH-COO-COOR''
b) treating directly the reaction mixture containing the mixed anhydride (III) with the amine of formula (IV)

   ₂HN-R-X (IV)

   wherein R and X have the above mentioned meanings, thereby obtaining the corresponding amides of formula (I).

The second process consists in reacting the α or β biotin ester of N-hydroxy-succinimide, in dimethylformamide at a temperature comprised between -10°C and 0°C, with the above mentioned amine (IV).

### DETAILED DESCRIPTION OF THE INVENTION

During the course of the instant description the compounds of formula (I) defined as β-derivatives and are characterized by having the substituent 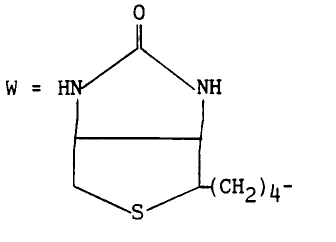
whereas those indicated with the definition "α-biotinyl" are those having 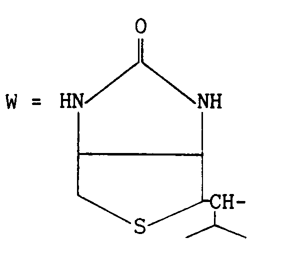
In the compounds of formula (I) preferred meanings for R, when this is defined as in class A: are -CH₂-CH₂-, 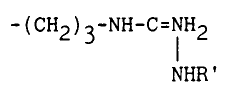
wherein R' has the above mentioned meanings,-(CH₂)₄-, 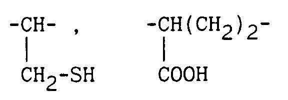
In the same compounds when R is defined as in class B, it is preferably cyclohexylene; in case R has the meanings given in class C it is preferably selected from 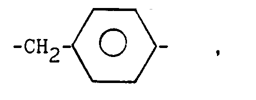 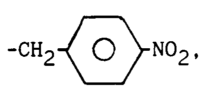
when R belongs to class D it is preferably

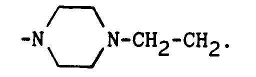

Particularly preferred compounds comprised in the general formula (I), which can be advantageously used in the therapeutical compositions according to the present inventions are the following:
- N-β-biotinyl-ethanolamine, namely the compound of formula (I) having R = -(CH₂)₂-, X = OH;
- N-β-biotinyl-N^{G}-methyl-L-arginine, namely the compound of formula (I) having 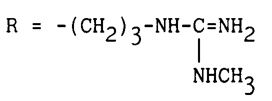 and X =COOH;
- N¹-β-biotinyl-N⁴-(2-hydroxyethyl)- piperazine, namely the compound of formula (I) having 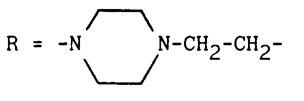 and X = OH;
- N-β-biotinyl-benzylamine, namely the compound of formula (I) having 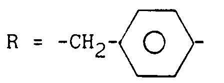 and X = H;
- N-β-biotinyl-4-amino-butanol, namely the compound of formula (I) having R = -(CH₂)₄-, and X = OH;
- N,N'bis-(β-biotinyl)-ethylendiamine, namely the compound of formula (I) having R = -(CH₂)₂-and 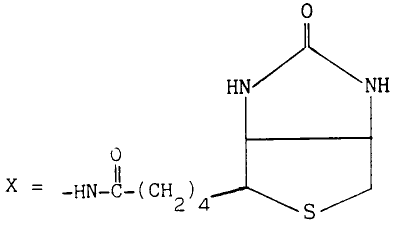
- N-β-biotinyl-L-serine, namely the compound of formula (I) having 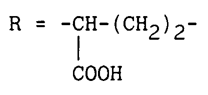 and X = OH;
- N-β-biotinyl-1-aminobutane, namely the compound of formula (I) having R = -(CH₂)₄-, and X = H;
- N-β-biotinyl-aminocyclohexane, namely the compound of formula (I) having R = cyclohexylene, and X = H;
- N-β-biotinyl-4-nitrobenzylamine, namely the compound of formula (I) having 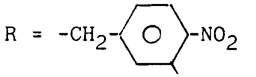 and X =H;
- N-β-biotinyl-L-arginine, namely the compound of formula (I) having 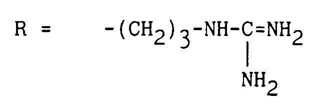 and X =COOH;
- N-β-biotinyl-L-cysteine, namely the compound of formula (I) having

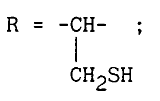

The biotinyl ethanolamine, is already known since it is utilized as intermediate to condense biotin to phosphoryl-oligonucleotides.

The "biotinised" oligonucleotides thus obtained are directly used as hybridization probe or for the preparation of longer probes for enzymatic reactions (T.Kempe et al" Chemical and enzymatic biotin labelling of oligodeoxyribonucleotides " Nucleic acid research 13(1): 45-47).

The amides of formula (I) according to the present invention are in particular able to control glucidic metabolism under dysmetabolic conditions, which can be correlated to the diabetic state and as such they can be used for non insulin-independent diabetes therapy alone or optionally in association with antidiabetic to be orally administered, and in insulin - dependent diabetes, optionally in association with insulin.

The derivative of formula (I) can be also advantageously used for the treatment of diabetes complicating diseases, as for example peripheral neuropathies, chronic invalidating pathologies.

The use of the derivatives of formula (I) alone or in association with other antidiabetic drugs (insulin or oral hypoglycemic drugs) has to be established on the base of the diabetes type and the seriousness pathology.

The use of the sole derivatives of general formula (I) is preferable under weak hyperglycemia conditions, due for example to aging, whereas the combined use of the compound of formula (I) and other antidiabetic drugs is advisable in case of clear and serious diabetic state, or in case of diabetes on genetic base.

In these cases the association may have the advantage of a control on glycemia, using lower dosages of conventionally used antidiabetic drugs, inclusive insulin, than the usual posology.

The compounds of formula (I) are preferably administered at dosages of from 5 to 20mg/die and the therapeutic regimen is of chronic type or at repeated cycles.

In any case the posology has to be established on the base of medical criteria, as for example seriousness pathology, the presence of accompanying pathologies, patient's weight and age and the administration type.

The therapeutical compositions of the present invention may be generally administered by oral or parenteral, preferably selected from subcutaneous and intramuscular route.

These compositions contain further to the active principle also pharmaceutically acceptable excipients or diluents.

The therapeutical compositions according to the present invention suitable to be parenterally administered are preferably in the form of aqueous and isotonic solution, whereas those suitable for the oral administration are in the form of lyophilized and granulated powders, tablets, dragees or capsules.

In both processes above cited the polar aprotic solvent is preferably dimethylformamide or tetrahydrofuran.

Triethylamine and tributylamine are preferably used as tertiary amine in both process.

In the first process, as the chloroformate isobutylchloroformate is preferably utilized, the temperature of step (a) is preferably comprised between -10°C and 0°C, and, the temperature of step (b) is preferably 0°C.

The product obtained with the processes of the present invention is preferably separated from the reaction mixture with conventional techniques such as lyophylization and filtration and are purified on anionic exchange resin or by inverse phase chromatography or by crystallization. The majority of the compounds of general formula (I) are soluble both in water and in organic solvents.

The following examples of preparation of the compounds of formula (I) of the present invention are reported for illustrative but not limitative purposes.

### Example 1: Preparation of N-β-biotinyl-ethanolamine

2.13 g triethylamine (21 mmoles) are added to 5.0 g of d-biotin (20.5 mmol) previously suspended in 80 ml of anhydrous tetrahydrofuran.

The suspension is then left at -10°C under stirring. 2.87 g isobutylchloroformate (21 mmol) are slowly added drop by drop in 30 min. under continuous stirring. The mixture is kept for 2 hours at -10 °C, and subsequently for 15 hours at 0 °C. 2.5 g ethanolamine are then slowly added drop by drop in 30 min..

The mixture is brought to room temperature and left under stirring for 6 hours. The suspension is filtered on gooch G3 filter and the filtrate is discarded, the precipitate is solubilized in 100 ml ethanol/water 1:1 and eluted on a column containing 50 ml anionic exchange resin (Amberlite IRA 400), generated under the acetate form. The eluate is concentrated under vacuum and the residue is crystallized from 70 ml of 80% ethanol. The product is then recovered by filtration, washed 3 times with 20 ml cool ethanol and then dried under high vacuum. The physical-chemical characteristics of the derivative obtained by the process described in the example 1 are:

| | |
|---|---|
| - physical state | white crystalline powder |
| - raw formula | C₁₂H₂₁N₃O₃S |
| - molecular weight | 287.32 |
| - elemental analysis | C= 50.16%; H= 7.37%; N=14.63%; O= 16.71%; S= 11.14% |
| - solubility in organic solvents | >10 mg/ml in DMSO |
| - solubility in water | >10mg/ml |
| - melting point | 172 °C |

### Example 2 -Preparation of N-β-biotinyl-N^{G}-methyl-L-arginine.

5.0 g D-biotin, (20.5 mmol) are suspended in 80 ml anhydrous DMF, which 7.8 g (42 mmol) tributylamine are added to; the resulting suspension is stirred at -10°C. 2.87 g isobutylchloroformate, corresponding to 21 mmol, are slowly added drop by drop in the space of 30 minutes, while maintaining the reaction mixture under continuous stirring.

The mixture is then left for 2 hours at -10°C, and successively for 15 hours at 0°C. 3.95 g N^{G}-methyl-L-arginine are then slowly added, the mixture is then stirred for 24 hours at 0°C, then at room temperature for further 6 hours. The solvent is evaporated from the reaction mixture under vacuum, the residue is diluted with water and purified by inverse phase chromatography, using a Lichrosorb RP18 resin column, and as the eluent a mixture water /methanol 80:20. The eluate fractions containing the pure product are then collected and evaporated to dryness; the residue is solubilized in 50 ml water and lyophilized.
Yield: 5.8g
The physical-chemical characteristics of the product N-β-biotinyl-N^{G}-methyl-L-arginine are the following:

| | |
|---|---|
| - physical state | white amorphous powder |
| - raw formula | C₁₇H₃₀N₆O₄S |
| - molecular weight | 414.53 |
| - elemental analysis | C= 49.26%; H= 7.29%; N=20.27%; O= 15.44%; S= 7.74% |
| - solubility in organic solvents | >10 mg/ml in DMSO |
| - solubility in water | >10mg/ml |
| - TLC | chloroform/ methanol/ water/ 28% NH₃: 65: 35: 5: 3 Rf= 0.22 |

### Example 2-A -Preparation of N-β-biotinyl-N^{G}-methyl-L-arginine.

7.0 g (20.5 mmol) N-succinimidyl-D-biotinate are suspended in 80 ml anhydrous DMF, which 2.13 g, triethylamine are added to, while maintaining the reaction mixture under continuous stirring at 0°C. 3.95 g N^{G}-methyl-L-arginine are then added and the mixture is stirred for 2 hours at 0°C, then at 45°C for further 4 hours. The desired product is then recovered and purified as described in Example 2.
The yield is 5.9 g.

### Example 3 N¹-β-biotinyl-N⁴-(2-hydroxyethyl) piperazine hydrochlorate preparation.

5.0 g D-biotin (20.5 mmol), are suspended in 80 ml anhydrous THF, which 2.13 g, (21 mmol) triethylamine are added to; the resulting suspension is stirred at -10°C. 2.87 g (21 mmol) isobutylchloroformate, are slowly added drop by drop in the space of 30 minutes, while maintaining the reaction mixture under continuous stirring . The mixture is left for 2 hours at -10°C and successively for 15 hours at 0°C. 3.0 g 1-(2-hydroxyethyl) piperazine are then slowly added drop by drop in the space of 30 minutes.

The mixture is then brought to room temperature and stirred for further 6 hours. The suspension thus obtained is then filtered and the filtrate is discarded; the solid precipitate is solubilized in 100 ml water and titrated with diluted HCl until pH 4.0 is reached and the solution is concentrated under vacuum by using a rotavapor. The residue is purified by inverse phase chromatography, using a Lichrosorb RP18 resin column, and as the eluent a mixture water /ethanol 90:10. The eluate fractions containing the pure product are then collected and evaporated to dryness; the residue is dried under high vacuum.
Yield: 6.2 g
The physical-chemical characteristics of the product N¹-biotinyl-N⁴-(2-hydroxyethyl)-piperazine hydrochlorate are the following:

| | |
|---|---|
| - physical state | white powder |
| - raw formula | C₁₆H₂₈N₄O₃S .HCl |
| - molecular weight | 392.95 |
| - elemental analysis | C= 48.91%; H= 7.44%; N=14.26%; O= 12.22%; S= 8.16%; Cl= 9.02%; |
| - solubility in organic solvents | >10 mg/ml in DMSO |
| - solubility in water | >10mg/ml |
| - TLC | chloroform/ methanol/ water/ 28% NH₃: 80: 25: 2: 1 Rf= 0.50 |

### Example 4 N-β-biotinyl-benzylamine preparation.

5.0 g (21 mmol) D-biotin, are suspended in 80 ml anhydrous THF, which 2.13 g, triethylamine are added to; the resulting suspension is stirred at -10°C. 2.87 g (21 mmol) isobutylchloroformate, are slowly added drop by drop in the space of 30 minutes, while maintaining the reaction mixture under continuous stirring. The mixture is maintained for 2 hours at -10°C, and successively for 15 hours at 0°C. 3.5 g benzylamine are then dropwise slowly added in the space of 30 minutes.

The mixture is then brought to room temperature and then left under stirring for further 6 hours. The solvent is then evaporated to dryness under vacuum and the obtained residue is crystallized from 120 ml water. The product is then recovered by filtration, washed three times with 20 ml cool water and finally dried under high vacuum.
Yield: 5.2 g
The physical-chemical characteristics of the product N-biotinyl-benzylamine are the following:

| | |
|---|---|
| - physical state | white crystalline powder |
| - raw formula | C₁₇H₂₃N₃O₂S |
| - molecular weight | 333.46 |
| - elemental analysis | C= 61.23%; H= 6.95%; N=12.60%; O= 9.60%; S= 9.62% |
| - solubility in organic solvents | >10 mg/ml in DMSO; >10mg/ml in ethanol. |
| - solubility in water | poorly soluble |
| - TLC | chloroform/ methanol/ water/ 28% NH₃: 80: 25: 2: 1 Rf= 0.69 |

### Example 5 N-β-biotinyl-4-amino-butanol preparation.

5.0 g D-biotin (20.5 mmol) are suspended in 80 ml anhydrous THF, which 2.13 g (21 mmol), triethylamine are added to; the resulting suspension is stirred at -10°C. 2.87 g (21 mmol) isobutylchloroformate, are slowly added drop by drop in the space of 30 minutes, while maintaining the reaction mixture under continuous stirring . The mixture is left for 2 hours at-10°C and successively for 15 hours at 0°C. 3.0 g 4-amino-butanol are then slowly added drop by drop.

The mixture is then brought to room temperature for further 6 hours. The suspension thus obtained is then filtered and the filtrate is discarded. The residue is purified by inverse phase chromatography, using a Lichrosorb RP18 resin column, and as the eluent a mixture water /ethanol 90:10. The eluate fractions containing the pure product are then collected and evaporated to dryness; the residue is dried under high vacuum.
Yield: 4.7 g
The physical-chemical characteristics of the product N-biotinyl-4-amino-butanl are the following:

| | |
|---|---|
| - physical state | white powder |
| - raw formula | C₁₄H₂₅N₃O₃S |
| - molecular weight | 315.44 |
| - elemental analysis | C= 53.31%; H= 7.99%; N=13.32%; O= 15.22%; S= 10.17%; |
| - solubility in organic solvents | >10 mg/ml in DMSO |
| - solubility in water | >5 mg/ml |
| - TLC | chloroform/ methanol/ water/ 28% NH₃: 80: 25: 2: 1 Rf= 0.45 |

### Example 6 N,N'bis-(β-biotinyl)-ethylendiamine preparation.

5.0 g D-biotin (20.5 mmol), are suspended in 80 ml anhydrous DMF, which 4.26 g (41 mmol), triethylamine are added to; the resulting suspension is stirred at -10°C. 2.87 g (21 mmol) isobutylchloroformate, corresponding to 21 mmol, are slowly added drop by drop in the space of 30 minutes, while maintaining the reaction mixture under continuous stirring . The mixture is left for 2 hours at-10°C and successively for 15 hours at 0°C. 0.6 g ethylendiamine are then slowly added drop by drop.

The mixture is then brought to room temperature and stirred for further 20 hours. The suspension thus obtained is then filtered and the filtrate is discarded. The precipitate is washed three times with 10 ml water and finally crystallized from a mixture water/ethanol 1:1. The product is recovered by filtration, washed three times with 20 ml cool ethanol and finally dried under high vacuum.
Yield: 3.7 g
The physical-chemical characteristics of the product N-N'-bis-(biotinyl)-ethylendiamine are the following:

| | |
|---|---|
| - physical state | white crystalline powder |
| - raw formula | C₂₂H₃₆N₆O₄S₂ |
| - molecular weight | 512.70 |
| - elemental analysis | C= 51.54%; H= 7.08%; N=16.39%; O= 12.48%; S= 12.51%; |
| - solubility in organic solvents | > 2 mg/ml in hot DMSO |
| - solubility in water | poorly soluble |
| - TLC | chloroform/ methanol/ water/ 28% NH₃: 80: 25: 2: 1 Rf= 0.36 |

### Example 7 N-β-biotinyl-L-serine preparation.

7.0 g (20.5 mmol) N-succinimidyl-D-biotinate are suspended in 80 ml anhydrous DMF, which 2.13 g, (21 mmol) triethylamine are added to, while maintaining the reaction mixture under continuous stirring at 0°C. 2.1 g L-serine are then added and the mixture is stirred for 2 hours at 0°C, then at 45°C for further 4 hours.

The solvent is evaporated under vacuum and the residue is diluted with cool water, which 22 ml 1N HCl are added to: the formed precipitate is separated by filtration, and the filtrate solution is discarded. The precipitate is purified by inverse phase chromathography, using a Lichrosorb RP18 resin column, and as the eluent a mixture water/ethanol 70:30. The eluate fractions containing the pure product are then collected and evaporated to dryness; the residue is dried under high vacuum.
Yield: 5.1 g
The physical-chemical characteristics of the product N-biotinyl-L-serine are the following:

| | |
|---|---|
| - physical state | white powder |
| - raw formula | C₁₃H₂₁N₃O₅S |
| - molecular weight | 331.40 |
| - elemental analysis | C= 47.11%; H= 6.39%; N=12.68%; O= 24.14%; S= 9.68%; |
| - solubility in organic solvents | >10 mg/ml in DMSO |
| - solubility in water | >10 mg/ml in 0.1M phosphate buffer at pH 7.4 |
| - TLC | chloroform/ methanol/ water/ 28% NH₃: 65: 35: 5: 3 Rf= 0.28 |

### Example 7-A N-β-biotinyl-L-serine preparation.

5.0 g (20.5 mmol) D-biotin, are suspended in 80 ml anhydrous DMF, which 7.8 g (42 mmol), tributylamine are added to; the resulting suspension is stirred at -10°C. 2.87 g (21 mmol) isobutylchloroformate, are slowly added drop by drop in the space of 30 minutes, while maintaining the reaction mixture under continuous stirring . The mixture is left for 2 hours at-10°C and successively for 15 hours at 0°C. 2.1 g L-serine are then slowly added drop by drop. The mixture is stirred for 24 hours at 0°C, then at room temperature for further 6 hours. The obtained product is then recovered and purified as described in example 7.
The yield is 5.2 g.

### Example 8 -Preparation of N-β-biotinyl-1-aminobutane.

5.0 g (20.5 mmol) D-biotin, are suspended in 80 ml anhydrous THF, which 2.13 g (21 mmol), triethylamine are added to; the resulting suspension is stirred at -10°C. 2.87 g isobutylchloroformate, (21 mmol) are slowly added drop by drop in the space of 30 minutes, while maintaining the reaction mixture under continuous stirring. The mixture is maintained for 2 hours at -10°C, and successively for 15 hours at 0°C. 2.0 g 1-aminobutane are then slowly added drop by drop in the space of 30 minutes.

The mixture is then brought to room temperature and then left under stirring for further 6 hours. The solvent is then evaporated to dryness under vacuum and the obtained residue is crystallized from 250 ml water. The product is then recovered by filtration, washed twice with 20 ml distilled water and finally dried under high vacuum.
Yield: 4.25 g
The physical-chemical characteristics of the product N-biotinyl-1 aminobutane are the following:

| | |
|---|---|
| - physical state | white crystalline powder |
| - raw formula | C₁₄H₂₅N₃O₂S |
| - molecular weight | 299.44 |
| - elemental analysis | C= 56.16%; H= 8.42%; N=14.03%; O= 10.69%; S= 10.71% |
| - solubility in organic solvents | >10 mg/ml in DMSO. |
| - solubility in water | poorly soluble |
| - TLC | chloroform/ methanol/ water/ 28% NH₃: 80: 25: 2: 1 Rf= 0.66 |

### Example 9 N-β-biotinyl-aminocyclohexane preparation.

7.0 g (20.5 mmol) N-succinimidyl-D-biotinate, are suspended in 80 ml anhydrous DMF, which 2.13 g (21 mmol) triethylamine are added to, while maintaining the reaction mixture under continuous stirring at 0°C. 2.0 g cyclohexylamine are then added and the mixture is stirred for 2 hours at 0°C, then at room temperature for further 14 hours. The reaction mixture is then cooled to 0°C, then filtered; the filtered portion is then discarded, whereas the precipitate is washed three times with 20 ml cool water and dried under high vacuum.
Yield: 4.57 g
The physical-chemical characteristics of the product N-biotinyl-aminocyclohexane are the following:

| | |
|---|---|
| - physical state | white powder |
| - raw formula | C₁₆H₂₇N₃O₂S |
| - molecular weight | 325.48 |
| - elemental analysis | C= 59.05%; H= 8.36%; N=12.91%; O= 9.83%; S= 9.85% |
| - solubility in organic solvents | >10 mg/ml in DMSO. |
| - solubility in water | poorly soluble |
| - TLC | chloroform/ methanol/ water/ 28% NH₃: 80: 25: 2: 1 Rf= 0.68 |

### Example 10 N-β-biotinyl-4-nitrobenzylamine preparation.

5.0 g (20.5 mmol) D-biotin are suspended in 80 ml anhydrous THF, which 2.13 g (21 mmol) triethylamine are added to; the resulting suspension is stirred at -10°C. 2.87 g isobutylchloroformate, (21 mmol) are slowly added drop by drop in the space of 30 minutes, while maintaining the reaction mixture under continuous stirring. The mixture is maintained for 2 hours at -10°C, and successively for 15 hours at 0°C. 3.77 g 4-nitrobenzylamine hydrochlorate are then dissolved in 50 ml cool water which 2 g K₂C0₃ is added to ; the solution is extracted three times with 50 ml ethyl acetate and the extracted portions are washed twice with 50 ml cool water, collected and evaporated to dryness. The obtained residue is diluted with 10 ml DMF and the obtained solution is slowly added drop by drop in the space of 30 minutes to the mixture previously prepared and maintained under continuous stirring at 0°C. The resulting mixture is then brought to room temperature and stirred for further 6 hours, then the solvent is evaporated to dryness under vacuum. The residue is then crystallized from 240 ml water, the product is separated by filtration, washed three times with 20 ml cool water and finally dried under high vacuum.
Yield: 5.47 g
The physical-chemical characteristics of the product N-biotinyl-4-nitrobenzylamine are the following:

| | |
|---|---|
| - physical state | yellowish crystalline powder |
| - raw formula | C₁₁H₂₂N₄O₄S |
| - molecular weight | 378.46 |
| - elemental analysis | C= 53.95%; H= 5.86%; N=14.81%; O= 16.91%; S= 8.47% |
| - solubility in organic solvents | >10 mg/ml in DMSO. |
| - solubility in water | poorly soluble |
| - TLC | chloroform/ methanol/ water/ 28% NH₃: 80: 25: 2: 1 Rf= 0.63 |

### Example 11 N-β-biotinyl-L-arginine preparation.

7.0 g (20.5 mmol) N-succinimidyl-D-biotinate are suspended in 80 ml anhydrous DMF, which 2.13 g (21 mmol), triethylamine are added to, while maintaining the reaction mixture under continuous stirring at 0°C. 3.83 g L-arginine are then added and the mixture is stirred for 2 hours at 0°C, then at 45°C for further 4 hours. The solvent is evaporated from the reaction mixture under vacuum, the residue is diluted with water and purified by inverse phase chromathography, using a Lichrosorb RP18 resin column, and as the eluent, a mixture water /ethanol 1:1. The eluate fractions containing the pure product are then collected and evaporated to dryness; the residue is solubilized in 50 ml water and lyophilized.
Yield: 5.6 g
The physical-chemical characteristics of the product N-β-biotinyl-L-arginine are the following:

| | |
|---|---|
| - physical state | white amorphous powder |
| - raw formula | C₁₆H₂₈N₆O₄S |
| - molecular weight | 400.51 |
| - elemental analysis | C= 47.98%; H= 7.05%; N=20.98%; O= 15.98%; S= 8.01% |
| - solubility in organic solvents | >10 mg/ml in DMSO |
| - solubility in water | >10mg/ml |
| - TLC | chloroform/ methanol/ water/ 28% NH₃: 65: 35: 5: 3 Rf= 0.12 |

### Example 12 N-β-biotinyl-L-cysteine preparation.

7.0 g (20.5 mmol) N-succinimidyl-D-biotinate are suspended in 80 ml anhydrous DMF, which 2.13 g (21 mmol) triethylamine are added to, while maintaining the reaction mixture under continuous stirring at 0°C. 2.42 g L-cysteine are then added and the mixture is stirred for 2 hours at 0°C, then at 45°C for further 4 hours.

The solvent is evaporated under vacuum and the residue is diluted with cool water, which 22 ml 1N HCl are added to: the formed precipitate is separated by filtration, and the filtrate is discarded. The obtained precipitate is purified by operating repeated washes in aqueous suspension at 45°C, and it is finally dried under high vacuum .
Yield: 4.4 g
The physical-chemical characteristics of the product N-biotinyl-L-cysteine are the following:

| | |
|---|---|
| - physical state | white powder |
| - raw formula | C₁₃H₂₁N₃O₄S₂ |
| - molecular weight | 347.46 |
| - elemental analysis | C= 44.94%; H= 6.09%; N=12.09%; O= 18.42%: S= 18.46%; |
| - solubility in organic solvents | >10 mg/ml in DMSO |
| - solubility in water | >10 mg/ml in 0.1M phosphate buffer at pH 7.4 |
| - TLC | chloroform/ methanol/ water/ 28% NH₃: 65: 35: 5: 3 Rf= 0.35 |

The derivatives are then tested in order to define their biologic activity in experimental conditions of hyperglycemia induced both by food absence and by diabetes induced by streptozotocin. Hepatic glucokinase activity is examined in the experimental condition of food absence. Glycemia, insulinemia and the levels of Aceto Acetate (Ac.Ac.) are examined in the diabetes induced by streptozotocin.

### Experimental diabetes induced by streptozotocin:

10 male Wistar rats (200 g) are divided into 4 groups, the first group is composed by the controls, to the remaining groups streptozotocin is injected by intraperitoneal route (80 mg/kg). The third group is furthermore treated with biotin (3 mg/kg) by intraperitoneal route, and the fourth group with biotinyl ethanolamide (3 mg/kg) again by intraperitoneal route. The treatment is repeated for three days, then a blood sample is taken for analysis on the fifth day from the initial treatment.

| Treatment | Glycemia mmol/l | Insulinemia mU/l | AcAc nmol/l |
|---|---|---|---|
| Controls | 6.1 ± 1.1 | 11.3 ± 1.1 | 0 |
| STZ* | 33.6 ± 2.4 | 7.81 ± 2.0 | 1.04 ± 0 |
| STX* + Biotin | 7.4 ± 0.9 | 25.1 ± 1.5 | 0 |
| STZ* + Biot.Et.Amide | 6.0 ± 0.5 | 23.5 ± 1.8 | 0 |

| | | | |
|---|---|---|---|
| *STZ = streptozotocin | | | |

Glycemia is normalized by the treatment with biotin and more consistently by biotinylethanolamine as shown in the above reported data; analogously on the insulinemia both the biotinylethanolamine and the biotin as such are active.

### Ex vivo hepatic glucokinasic activity

### EXPERIMENT A

This experiment is carried out following to Chauhan et al. process (J. Biol. Chem. 266, 10035, 1991), according to this process, hepatic glucokinase in the rat, which activity is depressed by food absence is reactivated by the biotin action. 15 Wistar rats weighing on average 250 g are divided into three groups, the first one represent the controls, the second one is treated by intraperitoneal route with biotin (3 mg/kg) and the third group with biotinylethanolamine (3 mg/kg) again by intraperitoneal route. Rats are treated after 24 hours without food and after 6 hours are sacrificed by decapitation.

Livers are homogenized in 5 volumes 50 mM Tris at pH 7, 0.15 M KCl, 5 mM EGTA, 4 mM MgCl₂, and 2 mM dithiothreitol and centrifugated at 100.000 x g for 60 minutes. The surnatant, frozen to -80 °C, is used for the analyses, after identifying proteins according to Bradford method (Anal. Biochem. 72, 248, 1976).

Glucokinase is dosed by spectrophotometry according to Dakshinamurti et al. (Can. J. Biochem. 46, 75, 1968) with the following results:

| Treatment | Glucokinasic activity (U./min/protein mg) |
|---|---|
| Controls | 2.20 ± 0.90 (mean ± stan. deviation) |
| biotin | 3.96 ± 0.70 |
| Biotinylethanolamine | 4.27 ± 0.45 |

The obtained differences are significant (t Student test; p = 0.01) and demonstrate that, after 6 hours, biotinylethanolamine is more active than biotin. Trials carried out with lesser interval between the administration and the sacrifice, show less pronounced differences. This bring to suppose that biotinylethanolamine derivative has a retarded action.

### EXPERIMENT B

Also this experiment, like the previous one, is carried out by following Chauhan et al. process (J. Biol. Chem. 266, 10035, 1991), according to this process, hepatic glucokinase in the rat, which activity is depressed by food absence and it is reactivated by the biotin action.

Sprague Dawley rat weighing on average 260 g left without food for 24 hours, are divided into 15 groups, each of 13 groups are respectively intraperitoneally treated with 2 mg/kg of one of the compounds of the present invention hereinbelow reported in Table 1 or with the same dosage of biotin, all these substances being previously dissolved in physiological solutions. The obtained results are compared with those of the two groups corresponding to normal rats feeded ad libitum and with untreated rats left without food for 24 hours, all the animals had free admission to water.

3 hours after the treatment the rats are sacrificed by cerebral displacement. Their livers are homogenized by Potter ( 8-9 strokes/800 rpm) in a volume 1:4 of 50 mM Tris at pH 7.4,0.15 M KCl, 5 mM EDTA, and 2 mM DTT. The homogenates are centrifugated at 100.000 x g for 60 minutes.Their proteic concentration is determined according to the above mentioned Bradford method (Anal. Biochem. 72, 248, 1976).

Glucokinase activity is evaluated according to Dakshinamurti et al. (Can. J. Biochem. 46, 75, 1968), and expressed as arbitrary units /proteic concentration of the samples.

The obtained results confirm that in the rats maintained without food for 24 hours a reduction on glucokinase activity is observed(3.078± 0.181 Arbitrary Units/mg proteins) if compared to the animal normally fed and that biotin is able to restore at least partially this enzymatic activity (3.662± 0.280 UA/mg proteins.

The compounds of the present invention proved to increase in a higher degree than biotin the above mentioned depressed activity ever since three hours from their administration as it clearly results from the following Table 1.

**TABLE 1**

| Effect of the compounds of the present invention on depressed glucokinase activity previously depressed by food absence, calculated as percentage of enzymatic activation with respect to that of the untreated group left without food (control group). | |
|---|---|
| administered compound | Glukokinase activity increase (%) |
| Biotin | 19 |
| N-β-biotinyl-N⁴-(2-hydroxyethyl)-piperazine | 28 |
| N-β-biotinyl-benzylamine | 28 |
| N-β-biotinyl-4-aminobutanol | 25 |
| N,N-'bis-(β-biotinyl)-ethylendiamine | 31 |
| N-β-biotinyl-L-serine | 33 |
| N-β-biotinyl-1-aminobutane | 25 |
| N-β-biotinyl-aminocyclohexane | 22 |
| N-β-biotinyl-4-nitrobenzylamine | 32 |
| N-β-biotinyl-L-arginine | 35 |
| N-β-biotinyl-N^{G}-methyl-L-arginine | 20 |
| N-β-biotinyl-L-cysteine | 34 |

In order to render evident the industrial applications of the present invention, some example are reported hereinbelow for illustrative but not limitative purposes. These compositions are given for illustrative purposes and are not to be considered limitative for the realization of the invention.

| Example 1: water-soluble tablets | |
|---|---|
| Biotinylethanolamine | 6 mg |
| Glycocoll | 62 mg |
| Sodium carboxymethylstarch | 22 mg |
| Polyethyleneglycol 6000 | 4 mg |
| Starch | 1 mg |
| Polyvinyl pyrrolidone | 5 mg |

| Example 2: granules sachets | |
|---|---|
| Biotinylethanolamine | 6 mg |
| Glycocoll | 964 mg |
| Polyvinyl pyrrolidone | 5 mg |
| Polyethyleneglycol 6000 | 5 mg |
| Peach flavouring essence | 20 mg |

| Example 3: 2 ml vials | |
|---|---|
| Biotinylethanolamine | 6 mg |
| Bibasic sodium phosphate 12 H₂O | 6 mg |
| Monobasic sodium phosphate | 0.5 mg |
| Sodium Chloride | 12 mg |
| Bidistilled water q.s. to | 2 ml |

## Claims

1. Amide derivatives of α or β biotin having general formula (I):
W-CONH-R-X (I)
wherein W is 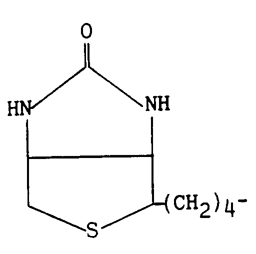 or 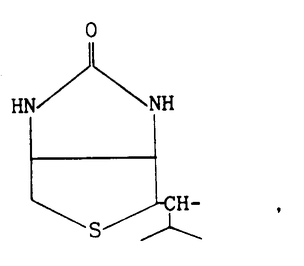 R is a bivalent radical of from 1 to 20 carbon atoms having the meanings defined in one of the following classes:
A) is a linear or branched alkylene radical, optionally substituted in the alkylene chain with at least one substituent selected from the group consisting of : -COOH, NH₂, 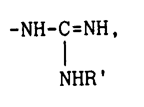 H, SH, -CO-, OH and wherein R' is methyl or H;
B) is a cycloalkylene of from 3 to 7 carbon atoms;
C) is an arylene or an arylalkylene radical optionally susbtituted on the aromatic ring with at least one NO₂ group;
D) is a piperazinylalkylene radical,
and X is selected from the group consisting of H, OH, COOH, H, 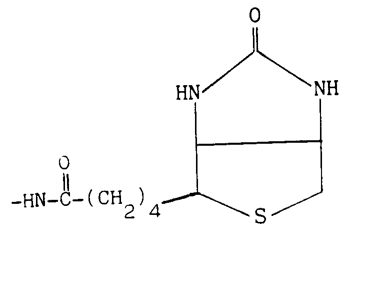 and 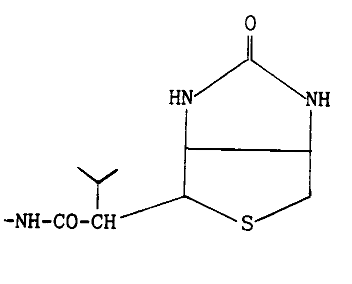 provided that when 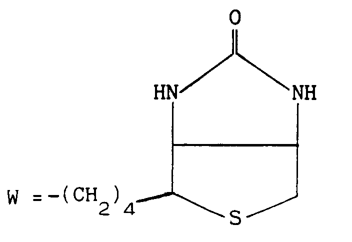 and R is -(CH₂)₂- X must be different from OH.

2. The amides as claimed in claim 1 wherein, when R is defined as in class A, it is preferably selected from the group consisting of -CH₂-CH₂-, 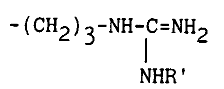 wherein R' has the above mentioned meanings,-(CH₂)₄-,

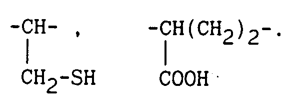

3. The amides as claimed in claim 1 wherein when R is defined as in class B it is cyclohexylene.

4. The amides as claimed in claim 1 wherein when R is defined as in class C it is preferably selected from the group consisting of:

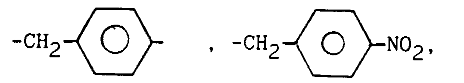

5. The amides as claimed in claim 1 wherein when R is defined as in class D it is

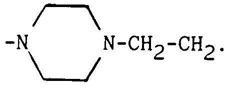

6. The amides as claimed in claim 2 selected from the group consisting of: N-β-biotinyl-N^{G}-methyl-L-arginine, N-β-biotinyl-4-amino-butanol, N,N'bis-(-β-biotinyl)-ethylediamine, N-β-biotinyl-L-serine, N-β-biotinyl-1-aminobutane, N-β-biotinyl-L-arginine, N-β-biotinyl-L-cysteine.

7. The amides as claimed in claim 3 consisting of N-β-biotinyl-aminocyclohexane.

8. The amides as claimed in claim 4 selected from the group consisting of: N-β-biotinyl-benzylamine, N-β-biotinyl-4-nitrobenzylamine.

9. The amides as claimed in claim 5 consisting of N¹-β-biotinyl-N⁴-(2-hydroxyethyl)- piperazine.

10. A process for preparing amide derivatives of α or β biotin having general formula (I):
W-CONH-R-X (I)
wherein W is 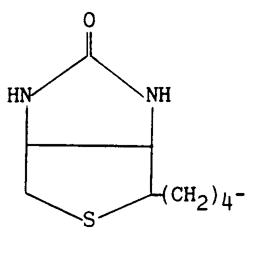 or 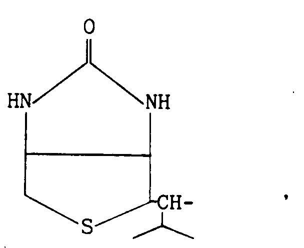 R is a bivalent radical of from 1 to 20 carbon atoms having the meanings defined in one of the following classes:
A) is a linear or branched alkylene radical, optionally substituted in the alkylene chain with at least one substituent selected from the group consisting of : -COOH, NH₂, 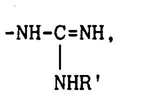 H, SH, -CO-, OH and wherein R' is methyl or H;
B) is a cycloalkylene of from 3 to 7 carbon atoms;
C) is an arylene or an arylalkylene radical optionally susbtituted on the aromatic ring with at least one NO₂ group;
D) is a piperazinylalkylene radical,
and X is selected from the group consisting of H, OH, COOH, H, 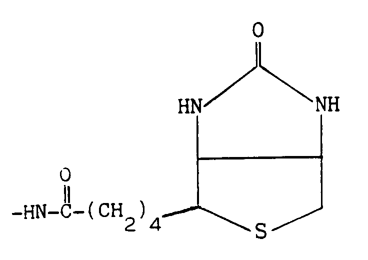 and 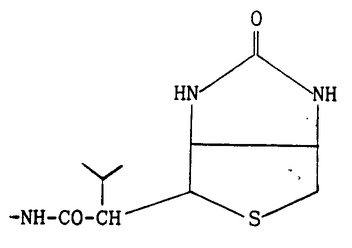 comprising the following steps:
a) treating biotin in a polar aprotic solvent, in the presence of a tertiary amine at a temperature lower than 0°C with alkylchloroformate (II):
Cl-COOR'' (II)
wherein R'' is a C₁-C₁₀ alkyl, thereby obtaining the corresponding mixed anhydride of formula (III);
W-NH-COO-COOR'' (III)
b) treating directly the reaction mixture containing the mixed anhydride (III) with the amine of formula (IV)
₂HN-R-X (IV)
wherein R and X have the above mentioned meanings, thereby obtaining the corresponding amides of formula (I).

11. A process for preparing amide derivatives of α or β biotin having general formula (I):
W-CONH-R-X (I)
wherein W is 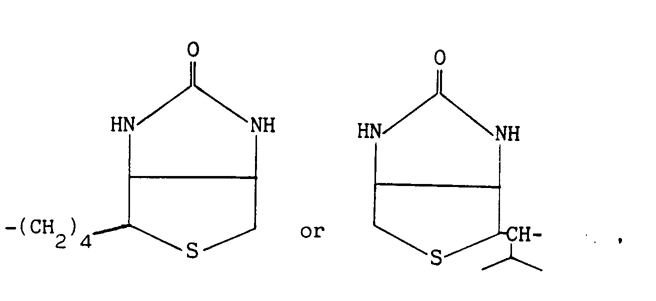 R is a bivalent radical of from 1 to 20 carbon atoms having the meanings defined in one of the following classes:
A) is a linear or branched alkylene radical, optionally substituted in the alkylene chain with at least one substituent selected from the group consisting of : -COOH, NH₂, 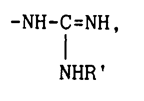 H, SH, -CO-, OH and wherein R' is methyl or H;
B) is a cycloalkylene of from 3 to 7 carbon atoms;
C) is an arylene or an arylalkylene radical optionally susbtituted on the aromatic ring with at least one NO₂ group;
D) is a piperazinylalkylene radical,
and X is selected from the group consisting of H, OH, COOH, H, 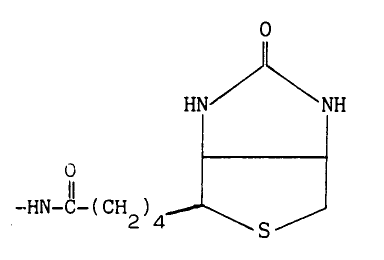 and 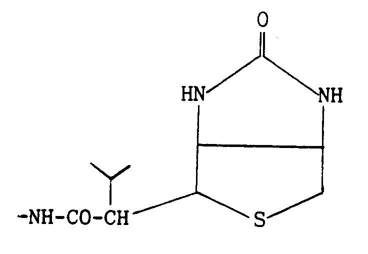 consisting in reacting the α or β biotin ester of N-hydroxy-succinimide, in dimethylformamide at a temperature comprised between -10°C and 0°C, with the amine (IV)
₂HN-R-X (IV)
wherein R and X have the above mentioned meanings.

12. Therapeutic compositions comprising as the active principle at least one of the amides of α or β biotin having general formula (I)
W-CONH-R-X (I)
wherein W is 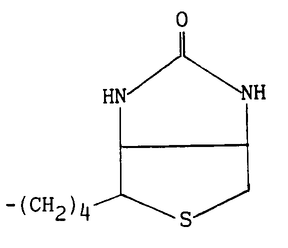 or 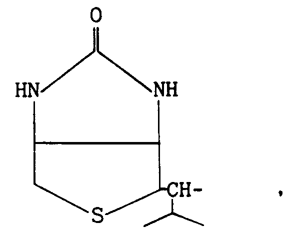 R is a bivalent radical of from 1 to 20 carbon atoms having the meanings defined in one of the following classes:
A) is a linear or branched alkylene radical, optionally substituted in the alkylene chain with at least one substituent selected from the group consisting of : -COOH, NH₂, 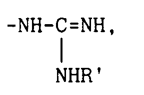 H, SH, -CO-, OH and wherein R' is methyl or H;
B) is a cycloalkylene of from 3 to 7 carbon atoms;
C) is an arylene or an arylalkylene radical optionally susbtituted on the aromatic ring with at least one NO₂ group;
D) is a piperazinylalkylene radical,
and X is selected from the group consisting of H, OH, COOH, H, 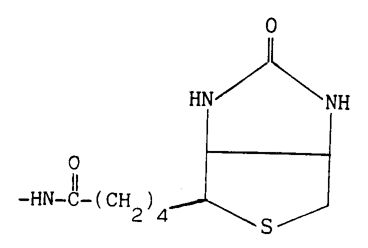 and 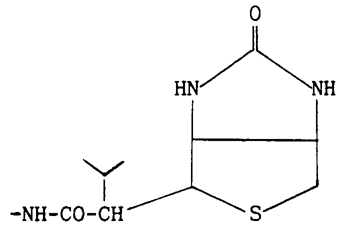 in combination with suitable excipients and/or diluents, for the treatment of diabetes and for the treatment and prevention of relative complicating diseases.

13. The therapeutic compositions as claimed in claim 12, for the treatment of insulin independent diabetes.

14. The therapeutic compositions as claimed in claim 13, optionally associated with hypoglycemic compositions orally administrable.

15. The therapeutic compositions as claimed in claim 12 for the treatment of insulin-dependent diabetes.

16. The therapeutic compositions as claimed in claim 15 optionally associated with injectable therapeutic compositions, containing as the active principle insulin.

17. The therapeutic composition as claimed in claim 12 for the treatment of diabetic complicating diseases selected from the group consisting of peripheral neuropathies and chronic invalidating pathologies.

18. The therapeutic compositions as claimed in claim 12 orally or parenterally administrable.

19. The therapeutic compositions as claimed in claim 12 wherein when R in formula (I) is defined as in class A, it is preferably selected from the group consisting of -CH₂-CH₂-, 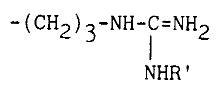 wherein R' has the above mentioned meanings,-(CH₂)₄-,

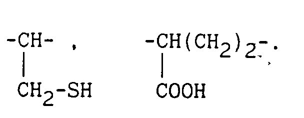

20. The therapeutic compositions as claimed in claim 12 wherein when R in formula (I) is defined as in class B, it is cyclohexylene.

21. The therapeutic compositions as claimed in claim 12 wherein when R in formula (I) is defined as in class C it is selected from the group consisting of:

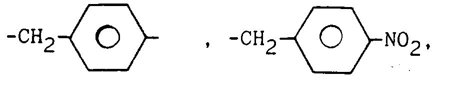

22. The therapeutic compositions as claimed in claim 12, wherein when R in formula (I) is defined as in class D, it is

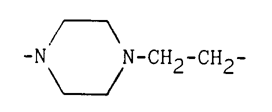

23. The therapeutic compositions as claimed in claim 19 wherein the compound of formula (I) are selected from the group consisting of: N-β-biotinylethanolamine, N-β-biotinyl-N^{G}-methyl-L-arginine, N-β-biotinyl-4-amino-butanol, N,N'bis-(-β-biotinyl)-ethylediamine, N-β-biotinyl-L-serine, N-β-biotinyl-1-aminobutane, N-β-biotinyl-L-arginine, N-β-biotinyl-L-cysteine.

24. The therapeutic compositions as claimed in claim 20 wherein the compound of formula (I) is N-β-biotinyl-aminocyclohexane.

25. The therapeutic compositions as claimed in claim 21 wherein the compound of formula (I) are selected from the group consisting of N-β-biotinyl-benzylamine, N-β-biotinyl-4-nitrobenzylamine.

26. The therapeutic compositions as claimed in claim 22 wherein the compound of formula (I) is N¹-β-biotinyl-N⁴-(2-hydroxyethyl)-piperazine.
